# EUROPEAN PATENT APPLICATION

(11) **EP 3 744 860 A1**
(43) Date of publication of application: **02.12.2020**
(21) Application number: 19815060.9
(22) Date of filing: 08.01.2019
(51) Int. Cl.: C12Q 1/6886, C12Q 1/6851, C12N 15/11

(54) **PRIMER AND PROBE SET FOR DIAGNOSIS, DETECTION, OR SCREENING OF COLORECTAL CANCER**

(30) Priority: 06.06.2018 CN 201810587088
(71) Applicant: Suzhou Versabio Technologies, Inc., Kunshan Suzhou, Jiangsu 215300 (CN)
(72) Inventor: ZHAO, Guodong, Suzhou, Jiangsu 215300 (CN)
(74) Representative: Grünecker Patent- und Rechtsanwälte PartG mbB
(86) International application number: PCT/CN2019/070750
(87) International publication number: WO 2019/233102

(57) **Abstract**

The present invention relates to the biomedical field, and relates to a primer and probe set for diagnosis, detection or screening of colorectal cancer, including amplification primers, a blocker primer, and a probe of SEPT9, and amplification primers, a blocker primer, and a probe of SDC2. A forward primer of SEPT9 is selected from any one of SEQ ID NOs: 1-25, a reverse primer of SEPT9 is selected from any one of SEQ ID NOs: 26-51, the blocker primer of SEPT9 is selected from any one of SEQ ID NOs: 52-67, and the probe of SEPT9 is selected from any one of SEQ ID NOs: 68-90. A forward primer of SDC2 is selected from any one of SEQ ID NOs: 91-117, a reverse primer of SDC2 is selected from any one of SEQ ID NOs: 118-143, the blocker primer of SDC2 is selected from any one of SEQ ID NOs: 144-156, and the probe of SDC2 is selected from any one of SEQ ID NOs: 157-174. Compared with the prior art, the present invention has the beneficial effect of simultaneously detecting methylation levels of SEPT9 and SDC2 with high sensitivity and specificity, for use in screening/detection/diagnosis of early-stage colorectal cancer.

## Description

### Technical Field

The present invention relates to the biomedical field, and relates to a primer and probe set for simultaneously detecting methylation levels of SEPT9 and SDC2 in a biological sample, for use in screening/detection/diagnosis of early-stage colorectal cancer.

### Background

Colorectal cancer is one of the most common malignant tumors. Cancer Statistics in China, 2015 showed that colorectal cancer ranked the top five among cancer types both in men and women, and colorectal cancer has been increasing year by year in the past decade. The current initial treatment staging proportion of colorectal cancer is: 15% in stage I, 20%-30% in stage II, 30%-40% in stage III, and 20%-25% in stage IV. In terms of survival, the 5-year survival rate of patients in stage I may reach more than 90%, while the survival rate of patients in stage IV is merely slightly greater than 10%. Therefore, application of early screening for colorectal cancer to achieve early diagnosis and early treatment is of great significance to improve the survival rate of patients and thus improve their overall health. Colonoscopy has many shortcomings as the gold standard for colorectal cancer screening, particularly in developing countries such as China. This method is highly demanding for operators. Moreover, as an invasive examination method, patients have great pain without anesthesia, leading to a low acceptance rate. At present, the screening rate is less than 3%. As a result, the early detection rate of colorectal cancer in China is much lower than that in developed countries.

At present, there are many methods for detecting colorectal cancer, such as fecal occult blood test, colonoscopy, computed tomography (CT), or fecal DNA detection. All of the above methods have been used to some extent, but they also have certain defects. Colonoscopy is currently the most sensitive method in colorectal cancer screening. However, this method requires professional operators and is expensive with low acceptance rates among patients, and has certain defects in early-stage colorectal cancer screening. The fecal occult blood test is a rapid detection method and is widely applied in colorectal cancer screening, but due to its low specificity, there are a large number of false positives, and the detection results are susceptible to interference from diet and drugs. Other methods, such as CT, may only detect cancers in the middle and advanced stages, so they cannot be used for early screening. The detection results of fecal DNA screening technology are easily interfered by impurities in feces. Moreover, the current fecal DNA detection method is to screen multiple biomarkers simultaneously, and due to the extremely high detection costs, it is currently difficult to popularize in developing countries like China. Furthermore, complicated operation steps also make it difficult to popularize in hospitals.

Abnormal DNA methylation is closely related to occurrence and persistence of many diseases, and especially, the research results in recent years show that DNA methylation plays an important role in induction and persistence of cancers, making it a good biomarker for many cancers.

SEPT9 is a very effective DNA methylation marker of cancer. Studies have shown that SETP9 presents significant expression differences in colorectal cancer tissues and normal tissues. Syndecan-2 (SDC2) protein generally functions as an integral membrane protein, and it is known to participate in cell proliferation, migration, and intercellular interactions through extracellular matrix protein receptors. The SDC2 gene is expressed in mesenchymal cells rather than epithelial cells in normal colon tissue. Studies have shown that patients with colorectal cancer show abnormal methylation of SDC2 at high frequency.

### Summary

To overcome the defects of the current early screening/detection/diagnosis method for colorectal cancer, the present invention provides a primer and probe set for simultaneously detecting methylation levels of SEPT9 and SDC2 genes in a biological sample. Early screening/detection/diagnosis of colorectal cancer is realized by extracting DNA from the biological sample, and detecting whether the biological sample contains methylated SEPT9 or SDC2 genes by using quantitative fluorescent PCR technology.

In order to solve the above technical problem, the present invention adopts the following technical solution: a primer and probe set for diagnosis, detection or screening of colorectal cancer includes amplification primers, a blocker primer, and a probe of SEPT9, and amplification primers, a blocker primer, and a probe of SDC2.

A forward primer of SEPT9 is selected from any one of SEQ ID NOs: 1-25, a reverse primer of SEPT9 is selected from any one of SEQ ID NOs: 26-51, the blocker primer of SEPT9 is selected from any one of SEQ ID NOs: 52-67, and the probe of SEPT9 is selected from any one of SEQ ID NOs: 68-90.

A forward primer of SDC2 is selected from any one of SEQ ID NOs: 91-117, a reverse primer of SDC2 is selected from any one of SEQ ID NOs: 118-143, the blocker primer of SDC2 is selected from any one of SEQ ID NOs: 144-156, and the probe of SDC2 is selected from any one of SEQ ID NOs: 157-174.

Further included is a reference gene primer probe set, where the reference gene is ACTB, a forward primer of ACTB is SEQ ID NO: 175, a reverse primer of ACTB is SEQ ID NO: 176, and a probe of ACTB is selected from SEQ ID NO: 177.

A detection object is a DNA sample obtained by extracting and purifying genomic DNA from a biological sample and then converting same using bisulfite, and carrying out re-purification (the sample serves as a DNA template of a PCR reaction system, and after the bisulfite conversion, a DNA template required for PCR is obtained through a conventional purification operation). The bisulfite is one or more of ammonium bisulfite, sodium bisulfite, and anhydrous sodium sulfate, and the concentration of the bisulfite is 8-10 M; the reaction conditions of the bisulfite conversion process are: DNA is added into a bisulfite solution of 8-10 M, and heated at 70-90°C for 30-60 min.

Detection of SEPT9 methylation, SDC2 methylation, and ACTB is performed simultaneously in one quantitative fluorescent PCR reaction, or a quantitative fluorescent PCR reaction is performed separately for one site (SEPT9, SDC2 or ACTB).

The blocker primer of SEPT9 and/or the blocker primer of SDC2 are modified using one of a phosphate group, C3, C9, and C18 at 3'-ends.

The present invention further provides an amplification system including the primer and probe set, where the specific components of the amplification system are:

| Components | Final concentration |
|---|---|
| PCR reaction buffer | 1 X |
| DNA polymerase | 0.08-0.2 U/µL |
| dNTPs mixed solution | 0.2-0.6 mM |
| Mg2+ | 2-8 mM |
| SEPT9 forward primer | 0.1-2 µM |
| SEPT9 reverse primer | 0.1-2 µM |
| SEPT9 blocker | 0.1-1.5 µM |
| SEPT9 probe | 0.01-0.2 µM |
| SDC2 forward primer | 0.1-1 µM |
| SDC2 reverse primer | 0.1-1 µM |
| SDC2 blocker | 0.1-1 µM |
| SDC2 probe | 0.01-0.2 µM |
| ACTB forward primer | 0.2 µM |
| ACTB reverse primer | 0.2 µM |
| ACTB probe | 0.1 µM |
| Deionized water | Add deionized water to 15 µL |
| DNA template | 15 µL |

The present invention further provides an application of the primer and probe set for detecting methylation of SEPT9 and SDC2 in a biological sample as a reagent in early screening/detection/diagnosis of colorectal cancer and precancerous lesions thereof, and further provides an application of the amplification system as a reagent in early screening/detection/diagnosis of colorectal cancer and precancerous lesions thereof.

Compared with the prior art, the present invention has the beneficial effect of simultaneously detecting methylation levels of SEPT9 and SDC2 with high sensitivity and specificity, for use in screening/detection/diagnosis of early-stage colorectal cancer.

### Brief Description of the Drawings

FIG. 1 is an amplification plot of 1000 copies, 100 copies, and 10 copies of methylated SEPT9 genes;
FIG. 2 is an amplification plot of 1000 copies, 100 copies, and 10 copies of methylated SDC2 genes;
FIG. 3 is a diagram showing the effects of a single SEPT9 primer probe set and a combined primer probe set of SEPT9 and SDC2 on detecting methylated SEPT9;
FIG. 4 is a diagram showing the effects of a single SDC2 primer probe set and a combined primer probe set of SEPT9 and SDC2 on detecting methylated SDC2;
FIG. 5 is a diagram showing the effects of a combined primer probe set of SEPT9 and SDC2 on detecting 17 cases of colorectal cancer tissues and corresponding cancer-adjacent tissues.

### Detailed Description

The present invention would be further described in detail with reference to the following embodiments, so that a person skilled in the art can implement the present invention with reference to the description text.

### Embodiment 1

A genomic sequence of a cell line whose SEPT9 and SDC2 genes are known to be fully methylated is taken as a DNA template; the DNA template is converted by heating at 70°C for 60 min in an ammonium bisulfite solution of 8 M, and then purified by an Axygen gel purification kit to obtain the converted DNA template which is then subjected to PCR reaction. The PCR reaction system is shown in Table 1, and the PCR reaction conditions are shown in Table 2.

**Table 1 PCR reaction system in embodiment 1**

| Components | Final concentration |
|---|---|
| PCR reaction buffer | 1 X |
| DNA polymerase | 0.1 U/µL |
| dNTPs mixed solution | 0.4 mM |
| Mg2+ | 2 mM |
| SEQ ID NO: 20 | 0.5 µM |
| SEQ ID NO: 38 | 0.5 µM |
| SEQ ID NO: 53 | 0.4 µM |
| SEQ ID NO: 79 | 0.1 µM |
| SEQ ID NO: 99 | 0.2 µM |
| SEQ ID NO: 127 | 0.2 µM |
| SEQ ID NO: 144 | 0.1 µM |
| SEQ ID NO: 167 | 0.1 µM |
| SEQ ID NO: 175 | 0.2 µM |
| SEQ ID NO: 176 | 0.2 µM |
| SEQ ID NO: 177 | 0.1 µM |
| Deionized water | Add deionized water to 15 µL |
| Converted DNA template | 15 µL |

3'-ends of SEQ ID NO: 53 and SEQ ID NO: 144 are modified using a phosphate group and C3, respectively. The reaction system consists of 15 µL of a reaction solution and 15 µL of the converted DNA template, with a total volume of 30 µL. 15 µL of the reaction solution is the remaining components except the converted DNA template. The following is the same.

**Table 2 PCR reaction conditions**

| Number of cycles | Temperature | Duration |
|---|---|---|
| 1 | 95°C | 30 min |
| 50 | 95°C | 10 s |
| | 56°C | 30 s |
| 1 | 40°C | 30 s |

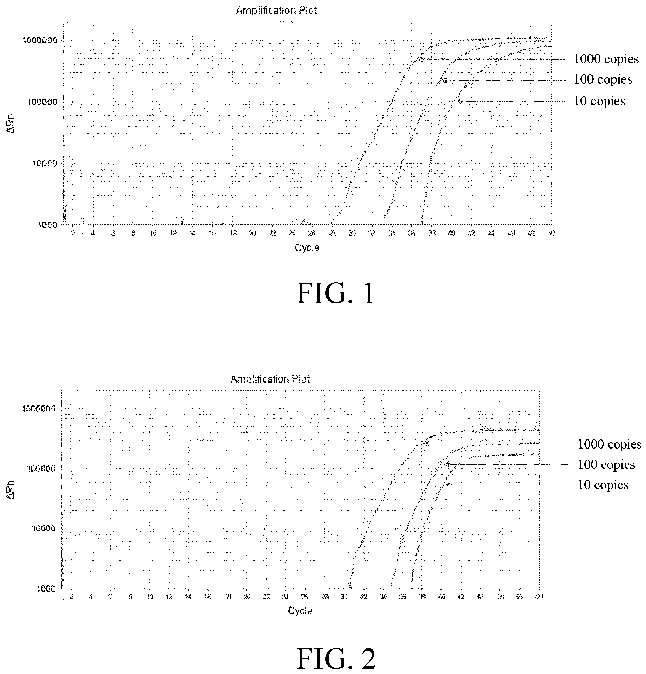

FIG. 1 shows amplification curves of 1000 copies, 100 copies, and 10 copies of methylated SEPT9 genes in embodiment 1. FIG. 2 shows amplification curves of 1000 copies, 100 copies, and 10 copies of methylated SDC2 genes in embodiment 1. As shown in FIGS. 1 and 2, the methylated SEPT9 and SDC2 have good amplification effects under the same reaction conditions, and the detection sensitivity reaches 10 copies.

### Embodiment 2

A genomic sequence of a cell line whose SEPT9 and SDC2 genes are known to be fully methylated is taken as a DNA template; the DNA template is converted by heating at 80°C for 40 min in an ammonium bisulfite solution of 9 M, and then purified by an Axygen gel purification kit to obtain the converted DNA template which is then subjected to PCR reaction. The components of the PCR reaction using the SEPT9 primer probe set alone are shown in Table 3. The components of the PCR reaction using the SDC2 primer probe set alone are shown in Table 4. The components of the PCR reaction using the combined primer probe set of SDC2 and SEPT9 are shown in Table 5. The PCR reaction conditions in embodiment 2 are shown in Table 6.

**Table 3 PCR components using SEPT9 primer probe set alone**

| Components | Final concentration |
|---|---|
| PCR reaction buffer | 1 X |
| DNA polymerase | 0.1 U/µL |
| dNTPs mixed solution | 0.4 mM |
| Mg2+ | 2 mM |
| SEQ ID NO: 3 | 1 µM |
| SEQ ID NO: 28 | 1 µM |
| SEQ ID NO: 52 | 0.8 µM |
| SEQ ID NO: 72 | 0.1 µM |
| SEQ ID NO: 175 | 0.2 µM |
| SEQ ID NO: 176 | 0.2 µM |
| SEQ ID NO: 177 | 0.1 µM |
| Deionized water | Add deionized water to 15 µL |
| Converted DNA template | 15 µL |

A 3'-end of SEQ ID NO: 52 is modified using C9.

**Table 4 PCR components using SDC2 primer probe set alone**

| Components | Final concentration |
|---|---|
| PCR reaction buffer | 1 X |
| DNA polymerase | 0.1 U/µL |
| dNTPs mixed solution | 0.4 mM |
| Mg2+ | 2 mM |
| SEQ ID NO: 91 | 0.4 µM |
| SEQ ID NO: 118 | 0.4 µM |
| SEQ ID NO: 144 | 0.2 µM |
| SEQ ID NO: 164 | 0.2 µM |
| SEQ ID NO: 175 | 0.2 µM |
| SEQ ID NO: 176 | 0.2 µM |
| SEQ ID NO: 177 | 0.1 µM |
| Deionized water | Add deionized water to 15 µL |
| Converted DNA template | 15 µL |

A 3'-end of SEQ ID NO: 144 is modified using C9.

**Table 5 PCR components using a combined primer probe set of SEPT9 and SDC2**

| Components | Final concentration |
|---|---|
| PCR reaction buffer | 1 X |
| DNA polymerase | 0.1 U/µL |
| dNTPs mixed solution | 0.4 mM |
| Mg2+ | 2 mM |
| SEQ ID NO: 3 | 1 µM |
| SEQ ID NO: 28 | 1 µM |
| SEQ ID NO: 52 | 0.8 µM |
| SEQ ID NO: 72 | 0.1 µM |
| SEQ ID NO: 91 | 0.4 µM |
| SEQ ID NO: 118 | 0.4 µM |
| SEQ ID NO: 144 | 0.2 µM |
| SEQ ID NO: 164 | 0.2 µM |
| SEQ ID NO: 175 | 0.2 µM |
| SEQ ID NO: 176 | 0.2 µM |
| SEQ ID NO: 177 | 0.1 µM |
| Deionized water | Add deionized water to 15 µL |
| Converted DNA template | 15 µL |

3'-ends of SEQ ID NO: 52 and SEQ ID NO: 144 are modified using C9.

**Table 6 PCR reaction conditions in embodiment 2**

| Number of cycles | Temperature | Duration |
|---|---|---|
| 1 | 95°C | 30 min |
| 50 | 95°C | 10 s |
| | 56°C | 30 s |
| 1 | 40°C | 30 s |

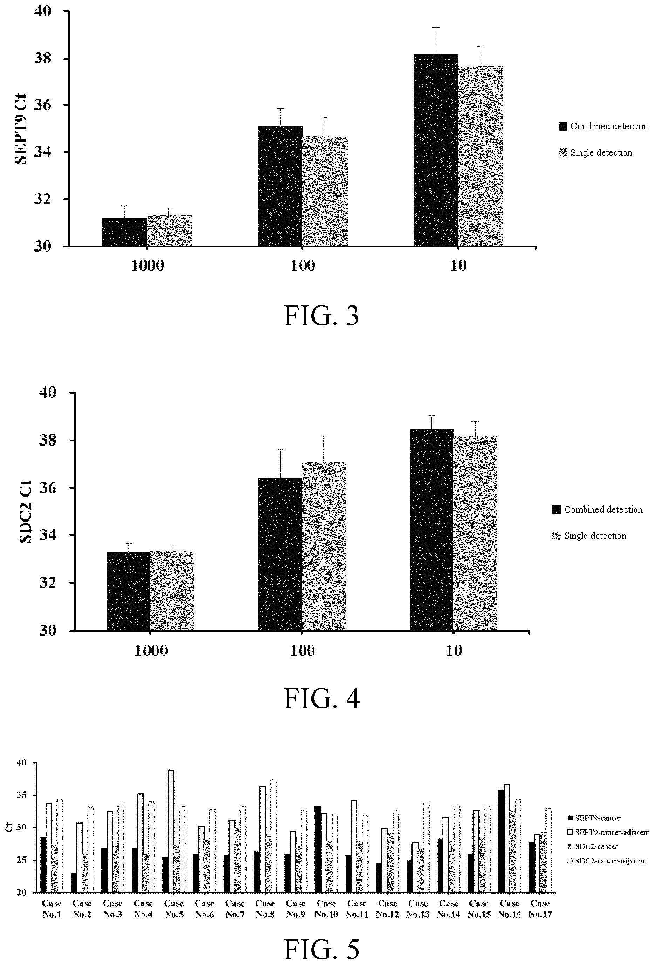

FIG. 3 is a schematic diagram showing the effects of a single SEPT9 primer probe set and a combined primer probe set of SEPT9 and SDC2 on detecting methylated SEPT9. FIG. 4 is a schematic diagram showing the effects of a single SDC2 primer probe set and a combined primer probe set of SEPT9 and SDC2 on detecting methylated SDC2. As can be seen in FIGS. 3 and 4, there is no significant difference between combined detection and separate detection of SDC2 and SEPT9.

### Embodiment 3

Colorectal cancer tissues and matched cancer-adjacent tissues are taken as detection objects. After DNA is extracted using a Qiagen DNeasy Blood & Tissue Kit, an ammonium bisulfite solution of 6 M, sodium bisulfite of 3 M, and anhydrous sodium sulfate of 1 M are used to be prepared into a bisulfite solution of 10 M. After DNA is converted, and then purified by an Axygen gel purification kit, the converted DNA template is obtained and then subjected to PCR reaction. The conversion condition is heating at 90°C for 30 min. The PCR reaction system is shown in Table 7, and the PCR reaction conditions are shown in Table 8.

**Table 7 PCR components in embodiment 3**

| Components | Final concentration |
|---|---|
| PCR reaction buffer | 1 X |
| DNA polymerase | 0.1 U/µL |
| dNTPs mixed solution | 0.4 mM |
| Mg2+ | 2 mM |
| SEQ ID NO: 1 | 1.5 µM |
| SEQ ID NO: 30 | 1.5 µM |
| SEQ ID NO: 55 | 1 µM |
| SEQ ID NO: 80 | 0.2 µM |
| SEQ ID NO: 96 | 0.5 µM |
| SEQ ID NO: 129 | 0.5 µM |
| SEQ ID NO: 156 | 0.2 µM |
| SEQ ID NO: 163 | 0.1 µM |
| SEQ ID NO: 175 | 0.2 µM |
| SEQ ID NO: 176 | 0.2 µM |
| SEQ ID NO: 177 | 0.1 µM |
| Deionized water | Add deionized water to 15 µL |
| Converted DNA template | 15 µL |

3'-ends of SEQ ID NO: 55 and SEQ ID NO: 156 are modified using a phosphate group and C18, respectively.

**Table 8 PCR reaction conditions in embodiment 3**

| Number of cycles | Temperature | Duration |
|---|---|---|
| 1 | 95°C | 30 min |
| 50 | 95°C | 10 s |
| | 56°C | 30 s |
| 1 | 40°C | 30 s |

FIG. 5 is a schematic diagram showing the effects of a combined primer probe set of SEPT9 and SDC2 on detecting 17 cases of colorectal cancer tissues and corresponding cancer-adjacent tissues. It can be seen from FIG. 5 that both SEPT9 and SDC2 can well distinguish colorectal cancer tissues from cancer-adjacent tissues, and therefore, SEPT9 and SDC2 can be used for early screening/detection/diagnosis of colorectal cancer.

### Embodiment 4

20 cases of colorectal serum samples of stages II-IV are collected with a volume of about 1.5 mL, labeled as a colorectal cancer group, and 56 cases of serum samples from patients without significant gastrointestinal diseases are collected with a volume of about 1 mL, labeled as a normal group. DNA is extracted by a cell-free DNA extraction kit of Suzhou VersaBio Technologies, Inc., then heated at 80°C for 45 min for conversion in an ammonium bisulfite solution of 9 M, and then purified by an Axygen gel purification kit to obtain a converted DNA template which is then subjected to PCR reaction. The PCR reaction system in embodiment 4 is shown in Table 9, and the reaction conditions are shown in Table 10. The effects of a combined primer probe set of SEPT9 and SDC2 on detecting SEPT9 and SDC2 methylation in cell-free DNA are shown in Table 11.

**Table 9 PCR components in embodiment 4**

| Components | Final concentration |
|---|---|
| PCR reaction buffer | 1 X |
| DNA polymerase | 0.1 U/µL |
| dNTPs mixed solution | 0.4 mM |
| Mg2+ | 2 mM |
| SEQ ID NO: 13 | 1.5 µM |
| SEQ ID NO: 50 | 1.5 µM |
| SEQ ID NO: 61 | 1 µM |
| SEQ ID NO: 74 | 0.2 µM |
| SEQ ID NO: 97 | 0.4 µM |
| SEQ ID NO: 125 | 0.4 µM |
| SEQ ID NO: 155 | 0.2 µM |
| SEQ ID NO: 165 | 0.1 µM |
| SEQ ID NO: 175 | 0.2 µM |
| SEQ ID NO: 176 | 0.2 µM |
| SEQ ID NO: 177 | 0.1 µM |
| Deionized water | Add deionized water to 15 µL |
| DNA template | 15 µL |

3'-ends of SEQ ID NO: 61 and SEQ ID NO: 155 are modified using C3.

**Table 10 PCR reaction conditions in embodiment 4**

| Number of cycles | Temperature | Duration |
|---|---|---|
| 1 | 95°C | 30 min |
| 50 | 95°C | 10 s |
| | 56°C | 30 s |
| 1 | 40°C | 30 s |

**Table 11 Effects of a combined primer probe set of SEPT9 and SDC2 in detecting SEPT9 and SDC2 methylation in cell-free DNA**

| Components | Detected gene | **Number of cases** | **Positive** | **Sensitivity** | **Specificity** |
|---|---|---|---|---|---|
| Colorectal cancer group | SEPT9 | 20 | 15 | 75% | / |
| | SDC2 | 20 | 18 | 90% | / |
| | SEPT9+SDC2 | 20 | 20 | 100% | / |
| Normal group | SEPT9 | 56 | 55 | / | 98% |
| | SDC2 | 56 | 51 | / | 91% |
| | SEPT9+SDC2 | 56 | 50 | / | 89% |

It can be seen from Table 11 that the combined detection of SEPT9 and SDC2 can significantly improve the sensitivity of colorectal cancer, and still has high specificity.

Sequences 1-177 of the present invention are shown in Table 12 below.

**Table 12 Primer and probe sequences**

| Remark | SED ID | Sequences |
|---|---|---|
| SEPT9 forward primer | SEQ ID NO: 1 | AAATAATCCCATCCAACTAC |
| | SED ID NO:2 | GTAGTTGGATGGGATTATTT |
| | SED ID NO:3 | AAATAATCCCATCCAACTACG |
| | SED ID NO:4 | CGTAGTTGGATGGGATTATTT |
| | SED ID NO:5 | AATAATCCCATCCAACTAC |
| | SED ID NO:6 | GTAGTTGGATGGGATTATT |
| | SED ID NO:7 | AATAATCCCATCCAACTACG |
| | SED ID NO:8 | CGTAGTTGGATGGGATTATT |
| | SED ID NO:9 | ATAATCCCATCCAACTACG |
| | SED ID NO:10 | CGTAGTTGGATGGGATTAT |
| | SED ID NO:11 | RAAATAATCCCATCCAACTA |
| | SED ID NO:12 | CRAAATAATCCCATCCAACTA |
| | SED ID NO:13 | TAGTTGGATGGGATTATTTYG |
| | SED ID NO:14 | RAAATAATCCCATCCAACT |
| | SED ID NO:15 | AGTTGGATGGGATTATTTY |
| | SED ID NO:16 | CRAAATAATCCCATCCAACT |
| | SED ID NO:17 | AGTTGGATGGGATTATTTYG |
| | SED ID NO:18 | CRAAATAATCCCATCCAAC |
| | SED ID NO:19 | GTTGGATGGGATTATTTYG |
| | SED ID NO:20 | CCACCTTCGAAATCCGAAAT |
| | SED ID NO:21 | ATTTCGGATTTCGAAGGTGG |
| | SED ID NO:22 | ATCCGAAATAATCCCATCCAA |
| | SED ID NO:23 | TTGGATGGGATTATTTCGGAT |
| | SED ID NO:24 | GATTTCGAAGGTGGGTGTTGGG |
| | SED ID NO:25 | CCCAACACCCACCTTCGAAATC |
| SEPT9 reverse primer | SED ID NO:26 | TCGTCGTTGTTTTTCGCGCGA |
| | SED ID NO:27 | TCGCGCGAAAAACAACGACGA |
| | SED ID NO:28 | CATAATAACTAATAAACAACRAATC |
| | SED ID NO:29 | GATTYGTTGTTTATTAGTTATTATG |
| | SED ID NO:30 | GATTYGTTGTTTATTAGTTATTAT |
| | SED ID NO:31 | ATAATAACTAATAAACAACRAATC |
| | SED ID NO:32 | GTTGTTTATTAGTTATTATGT |
| | SED ID NO:33 | ACATAATAACTAATAAACAAC |
| | SED ID NO:34 | GTAGGGTTCGGGTTTC |
| | SED ID NO:35 | GAAACCCGAACCCTAC |
| | SED ID NO:36 | TTCGTCGCTGTTTTTC |
| | SED ID NO:37 | GAAAAACAGCGACGAA |
| | SED ID NO:38 | GCGCGATTCGTTGTTTATTA |
| | SED ID NO:39 | TAATAAACAACGAATCGCGC |
| | SED ID NO:40 | TAGGGTTCGGGTTTCGT |
| | SED ID NO:41 | ACGAAACCCGAACCCTA |
| | SED ID NO:42 | GTAGGGTTCGGGTTTY |
| | SED ID NO:43 | RAAACCCGAACCCTAC |
| | SED ID NO:44 | TTYGTYGCTGTTTTTY |
| | SED ID NO:45 | RAAAAACAGCRACRAA |
| | SED ID NO:46 | GYGYGATTYGTTGTTTATTA |
| | SED ID NO:47 | TAATAAACAACRAATCRCRC |
| | SED ID NO:48 | TAGGGTTYGGGTTTYGT |
| | SED ID NO:49 | ACRAAACCCRAACCCTA |
| | SED ID NO:50 | YGATTYGTTGTTTATTAGTTATTATG |
| | SED ID NO:51 | CATAATAACTAATAAACAACRAATCR |
| SEPT9 blocker | SED ID NO:52 | TTGGATTTTGTGGTTAATGTGTAG |
| | SED ID NO:53 | TGTTGGATTTTGTGGTTAATGTGTAG |
| | SED ID NO:54 | GTTATTATGTTGGATTTTGTG |
| | SED ID NO:55 | GTTATTATGTTGGATTTTGTGGTTAATGTGTA |
| | SED ID NO:56 | GTTATTATGTTGGATTTTGTGGTTAATGTGT |
| | SED ID NO:57 | GTTATTATGTTGGATTTTGTGGTTAATGTG |
| | SED ID NO:58 | GTTATTATGTTGGATTTTGTGGT |
| | SED ID NO:59 | GTTATTATGTTGGATTTTGTGGTTAATGTGTAGAAAA |
| | SED ID NO:60 | TGTTTATTAGTTATTATGTTGGATTTTGTGGT |
| | SED ID NO:61 | TTATTATGTTGGATTTTGTGGTTAATGTGTAGAAA |
| | SED ID NO:62 | TTTATTAGTTATTATGTTGGATTTTGTGGT |
| | SED ID NO:63 | TTATTAGTTATTATGTTGGATTTTGTGGT |
| | SED ID NO:64 | TATTAGTTATTATGTTGGATTTTGTGGT |
| | SED ID NO:65 | ATTAGTTATTATGTTGGATTTTGTGGT |
| | SED ID NO:66 | TTAGTTATTATGTTGGATTTTGTGGT |
| | SED ID NO:67 | TAGTTATTATGTTGGATTTTGTGGT |
| SEPT9 probe | SED ID NO:68 | TTCGCGGTTAACGCGTAG |
| | SED ID NO:69 | CTACGCGTTAACCGCGAA |
| | SED ID NO:70 | ATTTCGCGGTTAACGCGT |
| | SED ID NO:71 | ACGCGTTAACCGCGAAAT |
| | SED ID NO:72 | GTTAACCGCGAAATCCGA |
| | SED ID NO:73 | TCGGATTTCGCGGTTAAC |
| | SED ID NO:74 | CGTTAACCGCGAAATCCGA |
| | SED ID NO:75 | TCGGATTTCGCGGTTAACG |
| | SED ID NO:76 | GCGTTAACCGCGAAATCCGA |
| | SED ID NO:77 | TCGGATTTCGCGGTTAACGC |
| | SED ID NO:78 | CGCGTTAACCGCGAAATCCGA |
| | SED ID NO:79 | CGTTAACCGCGAAATCCG |
| | SED ID NO:80 | GCGTTAACCGCGAAATCCGA |
| | SED ID NO:81 | CGCGTTAACCGCGAAATCCGA |
| | SED ID NO:82 | GTCGGATTTCGCGGTTAA |
| | SED ID NO:83 | GTCGGATTTCGCGGTTAAC |
| | SED ID NO:84 | GTCGGATTTCGCGGTTAACG |
| | SED ID NO:85 | GTCGGATTTCGCGGTTAACGC |
| | SED ID NO:86 | GTCGGATTTCGCGGTTAACGCG |
| | SED ID NO:87 | TCGGATTTCGCGGTTAAC |
| | SED ID NO:88 | CGGATTTCGCGGTTAACG |
| | SED ID NO:89 | CGGATTTCGCGGTTAACGC |
| | SED ID NO:90 | CGGATTTCGCGGTTAACGCG |
| SDC2 forward primer | SED ID NO:91 | AGTAAGAAGAGTTTTAGAGAGT |
| | SED ID NO:92 | ACTCTCTAAAACTCTTCTTACT |
| | SED ID NO:93 | GAAGAGTTTTAGAGAGTAGTT |
| | SED ID NO:94 | AACTACTCTCTAAAACTCTTC |
| | SED ID NO:95 | CGCGTTTTCGGGGCGTAG |
| | SED ID NO:96 | CTACGCCCCGAAAACGCG |
| | SED ID NO:97 | GGAGGAAGCGAGYGTTTT |
| | SED ID NO:98 | AAAACRCTCGCTTCCTCC |
| | SED ID NO:99 | TAGAAATTAATAAGTGAGAGG |
| | SED ID NO:100 | CCTCTCACTTATTAATTTCTA |
| | SED ID NO:101 | TTCGGGGCGTAGTTGCG |
| | SED ID NO:102 | CGCAACTACGCCCCGAA |
| | SED ID NO:103 | GCGTAGGAGGAGGAAGCGAGC |
| | SED ID NO:104 | GCTCGCTTCCTCCTCCTACGC |
| | SED ID NO:105 | GYGTAGGAGGAGGAAGCGAGC |
| | SED ID NO:106 | GCTCGCTTCCTCCTCCTACRC |
| | SED ID NO:107 | GYGTAGGAGGAGGAAGYGAGC |
| | SED ID NO:108 | GCTCRCTTCCTCCTCCTACRC |
| | SED ID NO:109 | GYGTAGGAGGAGGAAGYGAGY |
| | SED ID NO:110 | RCTCRCTTCCTCCTCCTACRC |
| | SED ID NO:111 | CGTAGGAGGAGGAAGCGA |
| | SED ID NO:112 | TCGCTTCCTCCTCCTACG |
| | SED ID NO:113 | YGTAGGAGGAGGAAGYGA |
| | SED ID NO:114 | TCRCTTCCTCCTCCTACR |
| | SED ID NO:115 | AATAAGTGAGAGGGCGTC |
| | SED ID NO:116 | GACGCCCTCTCACTTATT |
| | SED ID NO:117 | TTAATAAGTGAGAGGGTCG |
| SDC2 reverse primer | SED ID NO:118 | GTTTCGGATTCGTGTGCGC |
| | SED ID NO:119 | GCGCACACGAATCCGAAAC |
| | SED ID NO:120 | CGGATTCGTGTGCGCGGGTT |
| | SED ID NO:121 | AACCCGCGCACACGAATCCG |
| | SED ID NO:122 | GCGGTATTTTGTTTCGGATTCGT |
| | SED ID NO:123 | ACGAATCCGAAACAAAATACCGC |
| | SED ID NO:124 | CGTAATCGTTGCGGTATTT |
| | SED ID NO:125 | AAATACCGCAACGATTACG |
| | SED ID NO:126 | TTCGAGTTTTCGAGTTTGAG |
| | SED ID NO:127 | CTCAAACTCGAAAACTCGAA |
| | SED ID NO:128 | CGTAATCGTTGCRGTATTT |
| | SED ID NO:129 | AAATACYGCAACGATTACG |
| | SED ID NO:130 | CGTAATCRTTGCRGTATTT |
| | SED ID NO:131 | AAATACYGCAAYGATTACG |
| | SED ID NO:132 | CRTAATCRTTGCRGTATTT |
| | SED ID NO:133 | AAATACYGCAAYGATTAYG |
| | SED ID NO:134 | TTCGAGTTTTCRAGTTTGAG |
| | SED ID NO:135 | CTCAAACTYGAAAACTCGAA |
| | SED ID NO:136 | TTCRAGTTTTCRAGTTTGAG |
| | SED ID NO:137 | CTCAAACTYGAAAACTYGAA |
| | SED ID NO:138 | CGAGTTCGAGTTTTCGAGTTTG |
| | SED ID NO:139 | CAAACTCGAAAACTCGAACTCG |
| | SED ID NO:140 | CGAGTTCGAGTTTTCGAGTTT |
| | SED ID NO:141 | AAACTCGAAAACTCGAACTCG |
| | SED ID NO:142 | CGAGTTCGAGTTTTCGAGTT |
| | SED ID NO:143 | AACTCGAAAACTCGAACTCG |
| SDC2 blocker | SED ID NO:144 | GTAGGTGTAGGAGGAGGAAGTGAG |
| | SED ID NO:145 | CTCACTTCCTCCTCCTACACCTAC |
| | SED ID NO:146 | TTTGGGGTGTAGTTGTGGGTGG |
| | SED ID NO:147 | CCACCCACAACTACACCCCAAA |
| | SED ID NO:148 | TGAGTTTGAGTTTTTGAGTTTG |
| | SED ID NO:149 | CAAACTCAAAAACTCAAACTCA |
| | SED ID NO:150 | TTTGAGTTTGAGTTTTTGAG |
| | SED ID NO:151 | CTCAAAAACTCAAACTCAAA |
| | SED ID NO:152 | GGCGTAGTTGCGGGCGGCGG |
| | SED ID NO:153 | CCGCCGCCCGCAACTACGCC |
| | SED ID NO:154 | TGAGTGTTTTTGAGTTTTGAGTTTG |
| | SED ID NO:155 | AACTTGAAAACTTGAACTTGAAACT |
| | SED ID NO:156 | TGAGTGTTTTTGAGTTTTGAGTTTGAGTTTTTG |
| SDC2 probe | SED ID NO:157 | GGCGTAGGAGGAGGAAGCGAGCG |
| | SED ID NO:158 | CGCTCGCTTCCTCCTCCTACGCC |
| | SED ID NO:159 | GGCGTAGTTGCGGGCGGC |
| | SED ID NO:160 | GCCGCCCGCAACTACGCC |
| | SED ID NO:161 | TAGTTGCGGGCGGCGGG |
| | SED ID NO:162 | CCCGCCGCCCGCAACTA |
| | SED ID NO:163 | AGTTCGAGTTTTCGAGTTTGAG |
| | SED ID NO:164 | AACTCGAAAACTCGAACTCGAAACT |
| | SED ID NO:165 | AGTTTCGAGTTCGAGTTTTCGAGTT |
| | SED ID NO:166 | CTCAAACTCGAAAACTCGAACT |
| | SED ID NO:167 | GCGTAGGAGGAGGAAGCGA |
| | SED ID NO:168 | CGGGCGGCGGGAGTAGGCGT |
| | SED ID NO:169 | TCGCTTCCTCCTCCTACGC |
| | SED ID NO:170 | ACGCCTACTCCCGCCGCCCG |
| | SED ID NO:171 | CGTAGGAGGAGGAAGCGAGC |
| | SED ID NO:172 | GCTCGCTTCCTCCTCCTACG |
| | SED ID NO:173 | CGAGTTCGAGTTTTCGAGTTTGAG |
| | SED ID NO:174 | CTCAAACTCGAAAACTCGAACTCG |
| ACTB forward primer | SED ID NO:175 | GTGATGGAGGAGGTTTAGTAAGT |
| ACTB reverse primer | SED ID NO:176 | CCAATAAAACCTACTCCTCCCTTA |
| ACTB probe | SED ID NO:177 | CCACCACCCAACACACAATAACAAACAC |

Although the embodiments of the present invention have been disclosed as above, they are not limited merely to those set forth in the description and the embodiments, and they may be applied to various fields suitable for the present invention. For a person skilled in the art, other modifications may be easily achieved without departing from the general concept defined by the claims and their equivalents, and the present invention is not limited to the particular details.

## Claims

1. A primer and probe set for diagnosis, detection or screening of colorectal cancer, comprising amplification primers, a blocker primer, and a probe of SEPT9, and amplification primers, a blocker primer, and a probe of SDC2.

2. The primer and probe set for diagnosis, detection or screening of colorectal cancer according to claim 1, wherein a forward primer of SEPT9 is selected from any one of SEQ ID NOs: 1-25, a reverse primer of SEPT9 is selected from any one of SEQ ID NOs: 26-51, the blocker primer of SEPT9 is selected from any one of SEQ ID NOs: 52-67, and the probe of SEPT9 is selected from any one of SEQ ID NOs: 68-90.

3. The primer and probe set for diagnosis, detection or screening of colorectal cancer according to claim 1 or 2, wherein a forward primer of SDC2 is selected from any one of SEQ ID NOs: 91-117, a reverse primer of SDC2 is selected from any one of SEQ ID NOs: 118-143, the blocker primer of SDC2 is selected from any one of SEQ ID NOs: 144-156, and the probe of SDC2 is selected from any one of SEQ ID NOs: 157-174.

4. The primer and probe set for diagnosis, detection or screening of colorectal cancer according to claim 1, further comprising a reference gene primer probe set, wherein the reference gene is ACTB, a forward primer of ACTB is SEQ ID NO: 175, a reverse primer of ACTB is SEQ ID NO: 176, and a probe of ACTB is selected from SEQ ID NO: 177.

5. The primer and probe set for diagnosis, detection or screening of colorectal cancer according to claim 1, wherein a detection object is a DNA sample obtained by extracting and purifying genomic DNA from a biological sample and then converting same using bisulfite; the bisulfite is one or more of ammonium bisulfite, sodium bisulfite, and anhydrous sodium sulfate, and the concentration of the bisulfite is 8-10 M; the reaction conditions of the bisulfite conversion process are: DNA is added into a bisulfite solution of 8-10 M, and heated at 70-90°C for 30-60 min.

6. The primer and probe set for diagnosis, detection or screening of colorectal cancer according to claim 1, wherein detection of SEPT9 methylation, SDC2 methylation, and ACTB is performed simultaneously in one quantitative fluorescent PCR reaction, or a quantitative fluorescent PCR reaction is performed separately for one site.

7. The primer and probe set for diagnosis, detection or screening of colorectal cancer according to any one of claims 1 to 3, wherein the blocker primer of SEPT9 and/or the blocker primer of SDC2 are modified using one of a phosphate group, C3, C9, and C18 at 3'-ends.

8. An amplification system comprising the primer and probe set for diagnosis, detection or screening of colorectal cancer according to any one of claims 1 to 7, wherein the specific components of the amplification system are:
| Components | Final concentration |
|---|---|
| PCR reaction buffer | 1 X |
| DNA polymerase | 0.08-0.2 U/µL |
| dNTPs mixed solution | 0.2-0.6 mM |
| Mg2+ | 2-8 mM |
| SEPT9 forward primer | 0.1-2 µM |
| SEPT9 reverse primer | 0.1-2 µM |
| SEPT9 blocker | 0.1-1.5 µM |
| SEPT9 probe | 0.01-0.2 µM |
| SDC2 forward primer | 0.1-1 µM |
| SDC2 reverse primer | 0.1-1 µM |
| SDC2 blocker | 0.1-1 µM |
| SDC2 probe | 0.01-0.2 µM |
| ACTB forward primer | 0.2 µM |
| ACTB reverse primer | 0.2 µM |
| ACTB probe | 0.1 µM |
| Deionized water | Add deionized water to 15 µL |
| DNA template | 15 µL |

9. An application of the primer and probe set for diagnosis, detection or screening of colorectal cancer according to any one of claims 1 to 7 as a reagent in early screening/detection/diagnosis of colorectal cancer and precancerous lesions thereof.

10. An application of the amplification system according to claim 8 as a reagent in early screening/detection/diagnosis of colorectal cancer and precancerous lesions thereof.


Sequence Listing

<110>  SUZHOU VERSABIO TECHNOLOGIES, INC.

<120>  Primer and Probe Set for Diagnosis,
       Detection, or Screening of Colorectal Cancer

<130>  EP130352-FZMGpau

<140>  EP19815060.9
<141>  2019-01-08

<150>  CN201810587088
<151>  2018-06-06

<160>  177

<170>  SIPOSequenceListing 1.0

<210>  1
<211>  20
<212>  DNA
<213>  Homo sapiens

<400>  1
aaataatccc atccaactac                                                   20

<210>  2
<211>  20
<212>  DNA
<213>  Homo sapiens

<400>  2
gtagttggat gggattattt                                                   20

<210>  3
<211>  21
<212>  DNA
<213>  Homo sapiens

<400>  3
aaataatccc atccaactac g                                                 21

<210>  4
<211>  21
<212>  DNA
<213>  Homo sapiens

<400>  4
cgtagttgga tgggattatt t                                                 21

<210>  5
<211>  19
<212>  DNA
<213>  Homo sapiens

<400>  5
aataatccca tccaactac                                                    19

<210>  6
<211>  19
<212>  DNA
<213>  Homo sapiens

<400>  6
gtagttggat gggattatt                                                    19

<210>  7
<211>  20
<212>  DNA
<213>  Homo sapiens

<400>  7
aataatccca tccaactacg                                                   20

<210>  8
<211>  20
<212>  DNA
<213>  Homo sapiens

<400>  8
cgtagttgga tgggattatt                                                   20

<210>  9
<211>  19
<212>  DNA
<213>  Homo sapiens

<400>  9
ataatcccat ccaactacg                                                    19

<210>  10
<211>  19
<212>  DNA
<213>  Homo sapiens

<400>  10
cgtagttgga tgggattat                                                    19

<210>  11
<211>  20
<212>  DNA
<213>  Homo sapiens

<400>  11
raaataatcc catccaacta                                                   20

<210>  12
<211>  21
<212>  DNA
<213>  Homo sapiens

<400>  12
craaataatc ccatccaact a                                                 21

<210>  13
<211>  21
<212>  DNA
<213>  Homo sapiens

<400>  13
tagttggatg ggattattty g                                                 21

<210>  14
<211>  19
<212>  DNA

<213>  Homo sapiens

<400>  14
raaataatcc catccaact                                                    19

<210>  15
<211>  19
<212>  DNA
<213>  Homo sapiens

<400>  15
agttggatgg gattattty                                                    19

<210>  16
<211>  20
<212>  DNA
<213>  Homo sapiens

<400>  16
craaataatc ccatccaact                                                   20

<210>  17
<211>  20
<212>  DNA
<213>  Homo sapiens

<400>  17
agttggatgg gattatttyg                                                   20

<210>  18
<211>  19
<212>  DNA
<213>  Homo sapiens

<400>  18
craaataatc ccatccaac                                                    19

<210>  19
<211>  19
<212>  DNA
<213>  Homo sapiens

<400>  19
gttggatggg attatttyg                                                    19

<210>  20
<211>  20
<212>  DNA
<213>  Homo sapiens

<400>  20
ccaccttcga aatccgaaat                                                   20

<210>  21
<211>  20
<212>  DNA
<213>  Homo sapiens

<400>  21
atttcggatt tcgaaggtgg                                                   20

<210>  22
<211>  21

<212>  DNA
<213>  Homo sapiens

<400>  22
atccgaaata atcccatcca a                                                21

<210>  23
<211>  21
<212>  DNA
<213>  Homo sapiens

<400>  23
ttggatggga ttatttcgga t                                                21

<210>  24
<211>  22
<212>  DNA
<213>  Homo sapiens

<400>  24
gatttcgaag gtgggtgttg gg                                               22

<210>  25
<211>  22
<212>  DNA
<213>  Homo sapiens

<400>  25
cccaacaccc accttcgaaa tc                                               22

<210>  26
<211>  21
<212>  DNA
<213>  Homo sapiens

<400>  26
   tcgtcgttgt ttttcgcgcg a                                                 21

<210>  27
<211>  21
<212>  DNA
<213>  Homo sapiens

<400>  27
tcgcgcgaaa aacaacgacg a                                                21

<210>  28
<211>  25
<212>  DNA
<213>  Homo sapiens

<400>  28
cataataact aataaacaac raatc                                            25

<210>  29
<211>  25
<212>  DNA
<213>  Homo sapiens

<400>  29
gattygttgt ttattagtta ttatg                                            25

<210>  30

<211>  24
<212>  DNA
<213>  Homo sapiens

<400>  30
gattygttgt ttattagtta ttat                                              24

<210>  31
<211>  24
<212>  DNA
<213>  Homo sapiens

<400>  31
ataataacta ataaacaacr aatc                                              24

<210>  32
<211>  21
<212>  DNA
<213>  Homo sapiens

<400>  32
gttgtttatt agttattatg t                                                 21

<210>  33
<211>  21
<212>  DNA
<213>  Homo sapiens

<400>  33
acataataac taataaacaa c                                                 21

<210>  34
<211>  16
<212>  DNA
<213>  Homo sapiens

<400>  34
gtagggttcg ggtttc                                                       16

<210>  35
<211>  16
<212>  DNA
<213>  Homo sapiens

<400>  35
gaaacccgaa ccctac                                                       16

<210>  36
<211>  16
<212>  DNA
<213>  Homo sapiens

<400>  36
ttcgtcgctg tttttc                                                       16

<210>  37
<211>  16
<212>  DNA
<213>  Homo sapiens

<400>  37
gaaaaacagc gacgaa                                                       16

<210>  38
<211>  20
<212>  DNA
<213>  Homo sapiens

<400>  38
gcgcgattcg ttgtttatta                                                    20

<210>  39
<211>  20
<212>  DNA
<213>  Homo sapiens

<400>  39
taataaacaa cgaatcgcgc                                                    20

<210>  40
<211>  17
<212>  DNA
<213>  Homo sapiens

<400>  40
tagggttcgg gtttcgt                                                       17

<210>  41
<211>  17
<212>  DNA
<213>  Homo sapiens

<400>  41
acgaaacccg aacccta                                                       17

<210>  42
<211>  16
<212>  DNA
<213>  Homo sapiens

<400>  42
gtagggttcg ggttty                                                        16

<210>  43
<211>  16
<212>  DNA
<213>  Homo sapiens

<400>  43
raaacccgaa ccctac                                                        16

<210>  44
<211>  16
<212>  DNA
<213>  Homo sapiens

<400>  44
ttygtygctg ttttty                                                        16

<210>  45
<211>  16
<212>  DNA
<213>  Homo sapiens

<400>  45
raaaaacagc racraa                                                        16

<210>  46
<211>  20
<212>  DNA
<213>  Homo sapiens

<400>  46
gygygattyg ttgtttatta                                                    20

<210>  47
<211>  20
<212>  DNA
<213>  Homo sapiens

<400>  47
taataaacaa craatcrcrc                                                    20

<210>  48
<211>  17
<212>  DNA
<213>  Homo sapiens

<400>  48
tagggttygg gtttygt                                                       17

<210>  49
<211>  17
<212>  DNA
<213>  Homo sapiens

<400>  49
acraaacccr aacccta                                                       17

<210>  50
<211>  26
<212>  DNA
<213>  Homo sapiens

<400>  50
ygattygttg tttattagtt attatg                                             26

<210>  51
<211>  26
<212>  DNA
<213>  Homo sapiens

<400>  51
cataataact aataaacaac raatcr                                             26

<210>  52
<211>  24
<212>  DNA
<213>  Homo sapiens

<400>  52
ttggattttg tggttaatgt gtag                                               24

<210>  53
<211>  26
<212>  DNA
<213>  Homo sapiens

<400>  53

tgttggattt tgtggttaat gtgtag                                          26

<210>  54
<211>  21
<212>  DNA
<213>  Homo sapiens

<400>  54
gttattatgt tggattttgt g                                               21

<210>  55
<211>  32
<212>  DNA
<213>  Homo sapiens

<400>  55
gttattatgt tggattttgt ggttaatgtg ta                                   32

<210>  56
<211>  31
<212>  DNA
<213>  Homo sapiens

<400>  56
gttattatgt tggattttgt ggttaatgtg t                                    31

<210>  57
<211>  30
<212>  DNA
<213>  Homo sapiens

<400>  57
gttattatgt tggattttgt ggttaatgtg                                      30

<210>  58
<211>  23
<212>  DNA
<213>  Homo sapiens

<400>  58
gttattatgt tggattttgt ggt                                             23

<210>  59
<211>  37
<212>  DNA
<213>  Homo sapiens

<400>  59
gttattatgt tggattttgt ggttaatgtg tagaaaa                              37

<210>  60
<211>  32
<212>  DNA
<213>  Homo sapiens

<400>  60
tgtttattag ttattatgtt ggattttgtg gt                                   32

<210>  61
<211>  35
<212>  DNA
<213>  Homo sapiens

<400>  61
ttattatgtt ggattttgtg gttaatgtgt agaaa                                35

<210>  62
<211>  30
<212>  DNA
<213>  Homo sapiens

<400>  62
tttattagtt attatgttgg attttgtggt                                      30

<210>  63
<211>  29
<212>  DNA
<213>  Homo sapiens

<400>  63
ttattagtta ttatgttgga ttttgtggt                                       29

<210>  64
<211>  28
<212>  DNA
<213>  Homo sapiens

<400>  64
tattagttat tatgttggat tttgtggt                                        28

<210>  65
<211>  27
<212>  DNA
<213>  Homo sapiens

<400>  65
attagttatt atgttggatt ttgtggt                                         27

<210>  66
<211>  26
<212>  DNA
<213>  Homo sapiens

<400>  66
ttagttatta tgttggattt tgtggt                                          26

<210>  67
<211>  25
<212>  DNA
<213>  Homo sapiens

<400>  67
tagttattat gttggatttt gtggt                                           25

<210>  68
<211>  18
<212>  DNA
<213>  Homo sapiens

<400>  68
ttcgcggtta acgcgtag                                                   18

<210>  69
<211>  18
<212>  DNA
<213>  Homo sapiens

<400>  69
ctacgcgtta accgcgaa                                                    18

<210>  70
<211>  18
<212>  DNA
<213>  Homo sapiens

<400>  70
atttcgcggt taacgcgt                                                    18

<210>  71
<211>  18
<212>  DNA
<213>  Homo sapiens

<400>  71
acgcgttaac cgcgaaat                                                    18

<210>  72
<211>  18
<212>  DNA
<213>  Homo sapiens

<400>  72
gttaaccgcg aaatccga                                                    18

<210>  73
<211>  18
<212>  DNA
<213>  Homo sapiens

<400>  73
tcggatttcg cggttaac                                                    18

<210>  74
<211>  19
<212>  DNA
<213>  Homo sapiens

<400>  74
cgttaaccgc gaaatccga                                                   19

<210>  75
<211>  19
<212>  DNA
<213>  Homo sapiens

<400>  75
tcggatttcg cggttaacg                                                   19

<210>  76
<211>  20
<212>  DNA
<213>  Homo sapiens

<400>  76
gcgttaaccg cgaaatccga                                                  20

<210>  77
<211>  20
<212>  DNA

<213>  Homo sapiens

<400>  77
tcggatttcg cggttaacgc                                                    20

<210>  78
<211>  21
<212>  DNA
<213>  Homo sapiens

<400>  78
cgcgttaacc gcgaaatccg a                                                  21

<210>  79
<211>  18
<212>  DNA
<213>  Homo sapiens

<400>  79
cgttaaccgc gaaatccg                                                      18

<210>  80
<211>  20
<212>  DNA
<213>  Homo sapiens

<400>  80
gcgttaaccg cgaaatccga                                                    20

<210>  81
<211>  21
<212>  DNA
<213>  Homo sapiens

<400>  81
cgcgttaacc gcgaaatccg a                                                  21

<210>  82
<211>  18
<212>  DNA
<213>  Homo sapiens

<400>  82
gtcggatttc gcggttaa                                                      18

<210>  83
<211>  19
<212>  DNA
<213>  Homo sapiens

<400>  83
gtcggatttc gcggttaac                                                     19

<210>  84
<211>  20
<212>  DNA
<213>  Homo sapiens

<400>  84
gtcggatttc gcggttaacg                                                    20

<210>  85
<211>  21

<212>  DNA
<213>  Homo sapiens

<400>  85
gtcggatttc gcggttaacg c                                                21

<210>  86
<211>  22
<212>  DNA
<213>  Homo sapiens

<400>  86
gtcggatttc gcggttaacg cg                                               22

<210>  87
<211>  18
<212>  DNA
<213>  Homo sapiens

<400>  87
tcggatttcg cggttaac                                                    18

<210>  88
<211>  18
<212>  DNA
<213>  Homo sapiens

<400>  88
cggatttcgc ggttaacg                                                    18

<210>  89
<211>  19
<212>  DNA
<213>  Homo sapiens

<400>  89
cggatttcgc ggttaacgc                                                   19

<210>  90
<211>  20
<212>  DNA
<213>  Homo sapiens

<400>  90
cggatttcgc ggttaacgcg                                                  20

<210>  91
<211>  22
<212>  DNA
<213>  Homo sapiens

<400>  91
agtaagaaga gttttagaga gt                                               22

<210>  92
<211>  22
<212>  DNA
<213>  Homo sapiens

<400>  92
actctctaaa actcttctta ct                                               22

<210>  93

<211>  21
<212>  DNA
<213>  Homo sapiens

<400>  93
gaagagtttt agagagtagt t                                                21

<210>  94
<211>  21
<212>  DNA
<213>  Homo sapiens

<400>  94
aactactctc taaaactctt c                                                21

<210>  95
<211>  18
<212>  DNA
<213>  Homo sapiens

<400>  95
cgcgttttcg ggcgtag                                                     18

<210>  96
<211>  18
<212>  DNA
<213>  Homo sapiens

<400>  96
ctacgccccg aaaacgcg                                                    18

<210>  97
<211>  18
<212>  DNA
<213>  Homo sapiens

<400>  97
ggaggaagcg agygtttt                                                    18

<210>  98
<211>  18
<212>  DNA
<213>  Homo sapiens

<400>  98
aaaacrctcg cttcctcc                                                    18

<210>  99
<211>  21
<212>  DNA
<213>  Homo sapiens

<400>  99
tagaaattaa taagtgagag g                                                21

<210>  100
<211>  21
<212>  DNA
<213>  Homo sapiens

<400>  100
cctctcactt attaatttct a                                                21

<210>  101
<211>  17
<212>  DNA
<213>  Homo sapiens

<400>  101
ttcggggcgt agttgcg                                                      17

<210>  102
<211>  17
<212>  DNA
<213>  Homo sapiens

<400>  102
cgcaactacg ccccgaa                                                      17

<210>  103
<211>  21
<212>  DNA
<213>  Homo sapiens

<400>  103
gcgtaggagg aggaagcgag c                                                 21

<210>  104
<211>  21
<212>  DNA
<213>  Homo sapiens

<400>  104
gctcgcttcc tcctcctacg c                                                 21

<210>  105
<211>  21
<212>  DNA
<213>  Homo sapiens

<400>  105
gygtaggagg aggaagcgag c                                                 21

<210>  106
<211>  21
<212>  DNA
<213>  Homo sapiens

<400>  106
gctcgcttcc tcctcctacr c                                                 21

<210>  107
<211>  21
<212>  DNA
<213>  Homo sapiens

<400>  107
gygtaggagg aggaagygag c                                                 21

<210>  108
<211>  21
<212>  DNA
<213>  Homo sapiens

<400>  108
gctcrcttcc tcctcctacr c                                                 21

<210>  109
<211>  21
<212>  DNA
<213>  Homo sapiens

<400>  109
gygtaggagg aggaagygag y                                                21

<210>  110
<211>  21
<212>  DNA
<213>  Homo sapiens

<400>  110
rctcrcttcc tcctcctacr c                                                21

<210>  111
<211>  18
<212>  DNA
<213>  Homo sapiens

<400>  111
cgtaggagga ggaagcga                                                    18

<210>  112
<211>  18
<212>  DNA
<213>  Homo sapiens

<400>  112
tcgcttcctc ctcctacg                                                    18

<210>  113
<211>  18
<212>  DNA
<213>  Homo sapiens

<400>  113
ygtaggagga ggaagyga                                                    18

<210>  114
<211>  18
<212>  DNA
<213>  Homo sapiens

<400>  114
tcrcttcctc ctcctacr                                                    18

<210>  115
<211>  18
<212>  DNA
<213>  Homo sapiens

<400>  115
aataagtgag agggcgtc                                                    18

<210>  116
<211>  18
<212>  DNA
<213>  Homo sapiens

<400>  116

gacgccctct cacttatt                                                    18

<210>  117
<211>  19
<212>  DNA
<213>  Homo sapiens

<400>  117
ttaataagtg agagggtcg                                                   19

<210>  118
<211>  19
<212>  DNA
<213>  Homo sapiens

<400>  118
gtttcggatt cgtgtgcgc                                                   19

<210>  119
<211>  19
<212>  DNA
<213>  Homo sapiens

<400>  119
gcgcacacga atccgaaac                                                   19

<210>  120
<211>  20
<212>  DNA
<213>  Homo sapiens

<400>  120
cggattcgtg tgcgcgggtt                                                  20

<210>  121
<211>  20
<212>  DNA
<213>  Homo sapiens

<400>  121
aacccgcgca cacgaatccg                                                  20

<210>  122
<211>  23
<212>  DNA
<213>  Homo sapiens

<400>  122
gcggtatttt gtttcggatt cgt                                              23

<210>  123
<211>  23
<212>  DNA
<213>  Homo sapiens

<400>  123
acgaatccga aacaaaatac cgc                                              23

<210>  124
<211>  19
<212>  DNA
<213>  Homo sapiens

<400>  124
cgtaatcgtt gcggtattt                                                    19

<210>  125
<211>  19
<212>  DNA
<213>  Homo sapiens

<400>  125
aaataccgca acgattacg                                                    19

<210>  126
<211>  20
<212>  DNA
<213>  Homo sapiens

<400>  126
ttcgagtttt cgagtttgag                                                   20

<210>  127
<211>  20
<212>  DNA
<213>  Homo sapiens

<400>  127
ctcaaactcg aaaactcgaa                                                   20

<210>  128
<211>  19
<212>  DNA
<213>  Homo sapiens

<400>  128
cgtaatcgtt gcrgtattt                                                    19

<210>  129
<211>  19
<212>  DNA
<213>  Homo sapiens

<400>  129
aaatacygca acgattacg                                                    19

<210>  130
<211>  19
<212>  DNA
<213>  Homo sapiens

<400>  130
cgtaatcrtt gcrgtattt                                                    19

<210>  131
<211>  19
<212>  DNA
<213>  Homo sapiens

<400>  131
aaatacygca aygattacg                                                    19

<210>  132
<211>  19
<212>  DNA
<213>  Homo sapiens

<400>  132
crtaatcrtt gcrgtattt                                                    19

<210>  133
<211>  19
<212>  DNA
<213>  Homo sapiens

<400>  133
aaatacygca aygattayg                                                    19

<210>  134
<211>  20
<212>  DNA
<213>  Homo sapiens

<400>  134
ttcgagtttt cragtttgag                                                   20

<210>  135
<211>  20
<212>  DNA
<213>  Homo sapiens

<400>  135
ctcaaactyg aaaactcgaa                                                   20

<210>  136
<211>  20
<212>  DNA
<213>  Homo sapiens

<400>  136
ttcragtttt cragtttgag                                                   20

<210>  137
<211>  20
<212>  DNA
<213>  Homo sapiens

<400>  137
ctcaaactyg aaaactygaa                                                   20

<210>  138
<211>  22
<212>  DNA
<213>  Homo sapiens

<400>  138
cgagttcgag ttttcgagtt tg                                                22

<210>  139
<211>  22
<212>  DNA
<213>  Homo sapiens

<400>  139
caaactcgaa aactcgaact cg                                                22

<210>  140
<211>  21
<212>  DNA

<213>  Homo sapiens

<400>  140
cgagttcgag ttttcgagtt t                                                21

<210>  141
<211>  21
<212>  DNA
<213>  Homo sapiens

<400>  141
aaactcgaaa actcgaactc g                                                21

<210>  142
<211>  20
<212>  DNA
<213>  Homo sapiens

<400>  142
cgagttcgag ttttcgagtt                                                  20

<210>  143
<211>  20
<212>  DNA
<213>  Homo sapiens

<400>  143
aactcgaaaa ctcgaactcg                                                  20

<210>  144
<211>  24
<212>  DNA
<213>  Homo sapiens

<400>  144
gtaggtgtag gaggaggaag tgag                                             24

<210>  145
<211>  24
<212>  DNA
<213>  Homo sapiens

<400>  145
ctcacttcct cctcctacac ctac                                             24

<210>  146
<211>  22
<212>  DNA
<213>  Homo sapiens

<400>  146
tttggggtgt agttgtgggt gg                                               22

<210>  147
<211>  22
<212>  DNA
<213>  Homo sapiens

<400>  147
ccacccacaa ctacacccca aa                                               22

<210>  148
<211>  22

<212>  DNA
<213>  Homo sapiens

<400>  148
tgagtttgag tttttgagtt tg                                                22

<210>  149
<211>  22
<212>  DNA
<213>  Homo sapiens

<400>  149
caaactcaaa aactcaaact ca                                                22

<210>  150
<211>  20
<212>  DNA
<213>  Homo sapiens

<400>  150
tttgagtttg agtttttgag                                                   20

<210>  151
<211>  20
<212>  DNA
<213>  Homo sapiens

<400>  151
ctcaaaaact caaactcaaa                                                   20

<210>  152
<211>  20
<212>  DNA
<213>  Homo sapiens

<400>  152
ggcgtagttg cgggcggcgg                                                   20

<210>  153
<211>  20
<212>  DNA
<213>  Homo sapiens

<400>  153
ccgccgcccg caactacgcc                                                   20

<210>  154
<211>  25
<212>  DNA
<213>  Homo sapiens

<400>  154
tgagtgtttt tgagttttga gtttg                                             25

<210>  155
<211>  25
<212>  DNA
<213>  Homo sapiens

<400>  155
aacttgaaaa cttgaacttg aaact                                             25

<210>  156

<211>  33
<212>  DNA
<213>  Homo sapiens

<400>  156
tgagtgtttt tgagttttga gtttgagttt ttg                                    33

<210>  157
<211>  23
<212>  DNA
<213>  Homo sapiens

<400>  157
ggcgtaggag gaggaagcga gcg                                               23

<210>  158
<211>  23
<212>  DNA
<213>  Homo sapiens

<400>  158
cgctcgcttc ctcctcctac gcc                                               23

<210>  159
<211>  18
<212>  DNA
<213>  Homo sapiens

<400>  159
ggcgtagttg cgggcggc                                                     18

<210>  160
<211>  18
<212>  DNA
<213>  Homo sapiens

<400>  160
gccgcccgca actacgcc                                                     18

<210>  161
<211>  17
<212>  DNA
<213>  Homo sapiens

<400>  161
tagttgcggg cggcggg                                                      17

<210>  162
<211>  17
<212>  DNA
<213>  Homo sapiens

<400>  162
cccgccgccc gcaacta                                                      17

<210>  163
<211>  22
<212>  DNA
<213>  Homo sapiens

<400>  163
agttcgagtt ttcgagtttg ag                                                22

<210>  164
<211>  25
<212>  DNA
<213>  Homo sapiens

<400>  164
aactcgaaaa ctcgaactcg aaact                                          25

<210>  165
<211>  25
<212>  DNA
<213>  Homo sapiens

<400>  165
agtttcgagt tcgagttttc gagtt                                          25

<210>  166
<211>  22
<212>  DNA
<213>  Homo sapiens

<400>  166
ctcaaactcg aaaactcgaa ct                                             22

<210>  167
<211>  19
<212>  DNA
<213>  Homo sapiens

<400>  167
gcgtaggagg aggaagcga                                                 19

<210>  168
<211>  20
<212>  DNA
<213>  Homo sapiens

<400>  168
cgggcggcgg gagtaggcgt                                                20

<210>  169
<211>  19
<212>  DNA
<213>  Homo sapiens

<400>  169
tcgcttcctc ctcctacgc                                                 19

<210>  170
<211>  20
<212>  DNA
<213>  Homo sapiens

<400>  170
acgcctactc ccgccgcccg                                                20

<210>  171
<211>  20
<212>  DNA
<213>  Homo sapiens

<400>  171
cgtaggagga ggaagcgagc                                                20

<210>  172
<211>  20
<212>  DNA
<213>  Homo sapiens

<400>  172
gctcgcttcc tcctcctacg                                                    20

<210>  173
<211>  24
<212>  DNA
<213>  Homo sapiens

<400>  173
cgagttcgag ttttcgagtt tgag                                               24

<210>  174
<211>  24
<212>  DNA
<213>  Homo sapiens

<400>  174
ctcaaactcg aaaactcgaa ctcg                                               24

<210>  175
<211>  23
<212>  DNA
<213>  Homo sapiens

<400>  175
gtgatggagg aggtttagta agt                                                23

<210>  176
<211>  24
<212>  DNA
<213>  Homo sapiens

<400>  176
ccaataaaac ctactcctcc ctta                                               24

<210>  177
<211>  28
<212>  DNA
<213>  Homo sapiens

<400>  177
ccaccaccca acacacaata acaaacac                                           28